# EUROPEAN PATENT SPECIFICATION

(11) **EP 0 728 453 B1**
(45) Date of publication and mention of the grant of the patent: **07.05.2003**
(21) Application number: 96102769.5
(22) Date of filing: 23.02.1996
(51) Int. Cl.: A61F 6/04

(54) **Improvements on latex prophylactic**
Prophylaktikum aus Latex
Dispositif prophylactique de latex

(30) Priority: 24.02.1995 AR 33114595
(43) Date of publication of application: 28.08.1996
(73) Proprietor: Kopelowicz, Alberto, 1650 San Martin-Buenos Aires (AR)
(72) Inventor: Kopelowicz, Alberto, 1650 San Martin-Buenos Aires (AR)
(74) Representative: Canela Giménez, Teresa

(56) References cited:
- US-A- 5 176 152
- US-A- 5 284 159
- SOVIET PATENT ABSTRACTS Section PQ, Week 9505 3 March 1995 Derwent Publications Ltd., London, GB; Class P3, AN 95-034521 XP002025606 & SU 2 012 290 C (VOKHMYANIN, V.G.)

## Description

The present invention refers to improvements on latex prophylactics and the purpose of which is constituted by a certain and constructive arrangement, through which an increase in the resistence of the already known prophylactics is obtained, increasing its safety and efficiency, decreasing the risks of breakages, with the outstanding advantages of economic and practical order which shall be put in evidence in the course of the present specification.

The devices of prophylactics or contraceptives generally manufactured in latex or elastomeric material similar in a form such that be conveniently to use as it be convenient to cover the penis are already known, avoiding the communication of the aqueous humor in the sexual relation and as a consequence, the transmission of a plurality of diseases that seriously flagellates the world population and taking into consideration that said preservatives are preferably manufactured in an elastomeric material of the latex type or the like, present the serious disadvantage of its spontaneous breakage, present the serious unconveniency due to its generally, longitudinal stretching, so as its use becoming extremely risky in critical situations, this undesirable effects has been extremely surpassed. These undesired effects are overcome by the latex prophylactic according to claim 1, whereby the elasticity and adaptation and adjustment of the preservative transverse to its longitudinal direction and an inelasticity in the longitudinal direction so as to not prejudice its adaptability to provide a total resistence against breakage and split without affecting the adhesion or adaptability and without requiring more thickness in the walls of the preservative which may provoke irritation due to its use.

In order that the present invention be clearly understood and easily taking into operation, one preferred embodiment is illustrated in the drawings in which:

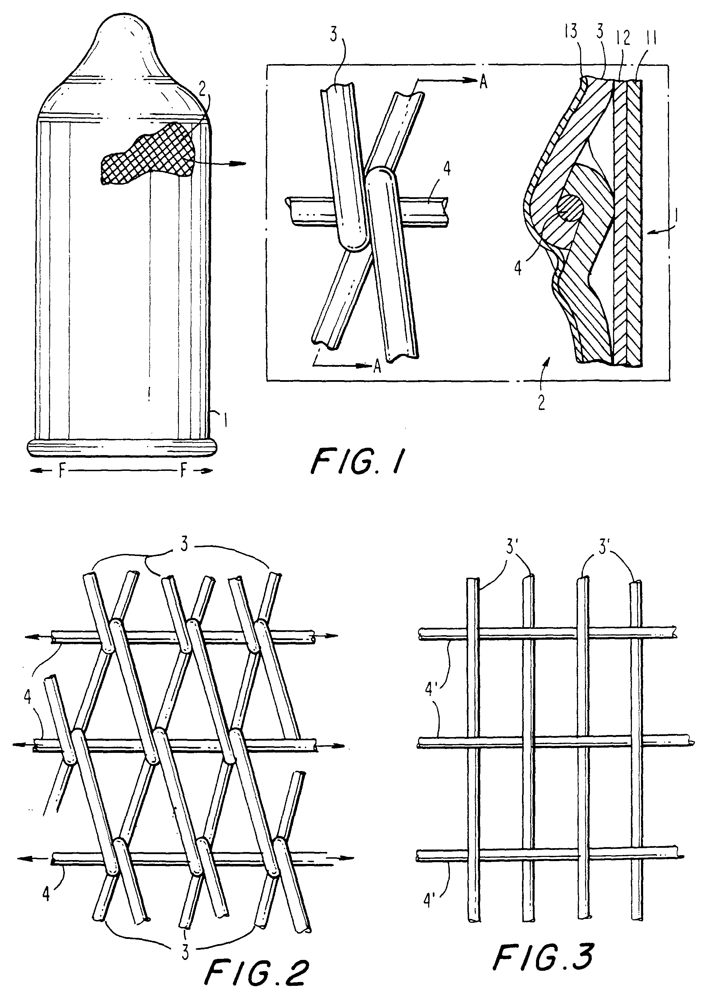

FIGURE 1: A perspective view of the improved prophylactic object of the present invention, showing a window detail of the incorporated improvement showing in detail a window of its improvement incorporated and of its structural configuration in elevation and a warp weft section through "A" "A" section.
FIGURE 2: Is a view of the incorporated fabric, showing the elastomeric filaments of the fabric and its elasticity; of the fabric warp and its elasticity sense direction and the rigid filaments of the weft, interweaving on the above-mentioned warp which are incorporated in the improvement illustrated in Figure 1.
FIGURE 3: Show a variant of the improvement illustrated in Figure 1, in the construction of which the elastomeric filaments of the warp, showing its expansion sense as well as the unelastic longitudinal filaments which in orthogonal and interweaved of the elastomeric filaments of the warp showing its expansion direction, as well as the unelastic longitudinal filaments that constitute the weft in the fabric of the improvement.

In said figures, the same reference figures indicate equal or similar parts.

Now, having been illustrated the improvement of the present invention we can appreciate that in the illustrated preservative construction (1) in the window opening are shown: the incorporated fabric (2), the fabric of which is constituted by an unelastic extremely thin filament (3) which constitute the weft connected to extremely thin elastomeric filaments (4) that constituted the warp of the fabric incorporated to the prophylactic, same being incorporated in the following manner: a) A base latex layer is shaped (11) on which a second layer being also of latex a second layer is arranged (12) and once both layers being consolidated it is superpossed to same, such as is illustrated in detail in Figure 1 the fabric (2) base of the present improvement to the basic body of the prophylactic being (2) in solidary to the set, through the coating of the assembly for a last and extremely thin latex layer (13) with which in definitive form is finally shaped, the prophylactic object of this invention, being appreciated the simple and effective manner for the obtainment of same, since that the fabric shaped by the weft (3) and the warp (4) it shall be allowed an elastic adjustment of the improved unit in radial direction to the prophylactic axis, following the deformation in the direction illustrated by arrows f-f', avoiding the stretching in the longitudinal direction of the prophylactic with which practically dissapear the possibilities of producing cracks or the breaking of same, since the longitudinal stretching is precisely the cause that most frequently produce same.

## Claims

1. Latex prophylactic (1) having a tubular structure with a closed distal end and an open proximal end, **characterised in**
a fabric (2) incorporated within the tubular structure of the prophylactic (1), said fabric (2) being constituted by an inelastic weft (3) extending in the longitudinal direction of the prophylactic (1), and an elastomeric warp (4) extending transversely with respect to the longitudinal direction of the prophylactic (1).

2. Latex prophylactic (1) as claimed in claim 1, **characterized in that** said fabric (2) is interpolated between two of a plurality of latex layers (11, 12, 13) forming the tubular structure of the prophylactic (1).

## Patentansprüche

1. Latexprophylaktikum (1), das eine röhrenförmige Struktur mit einem geschlossenen distalen Ende und einem offenen proximalen Ende hat, **gekennzeichnet durch**
ein in die röhrenförmige Struktur des Prophylaktikums (1) integriertes textiles Flächengebilde (2) gebildet aus einem in Längsrichtung des Prophylaktikums (1) verlaufenden unelastischen Schuss (3) und einer in Bezug auf die Längsrichtung des Prophylaktikums (1) quer verlaufenden elastomeren Kette (4).

2. Latexprophylaktikum (1) nach Anspruch 1, **dadurch gekennzeichnet, daß** das textile Flächengebilde (2) zwischen zwei einer Vielzahl von Latexschichten (11, 12, 13) eingelagert ist, die die röhrenförmige Struktur des Prophylaktikums (1) bilden.

## Revendications

1. Dispositif prophylactique en latex (1) ayant une structure tubulaire avec une extrémité distale fermée et une extrémité proximale ouverte, **caractérisé en ce qu'**un tissu (2) est incorporé dans la structure tubulaire du dispositif prophylactique (1), ledit tissu (2) étant constitué d'une trame non élastique (3) s'étendant dans la direction longitudinale du dispositif prophylactique (1) et d'une chaîne élastomère (4) s'étendant transversalement par rapport à la direction longitudinale du dispositif prophylactique (1).

2. Dispositif prophylactique en latex (1) selon la revendication 1, **caractérisé en ce que** ledit tissu (2) est inséré entre deux couches d'une pluralité de couches en latex (11, 12, 13) formant la structure tubulaire du dispositif prophylactique (1).
